# EUROPEAN PATENT SPECIFICATION

(11) **EP 3 824 907 B1**
(45) Date of publication and mention of the grant of the patent: **17.09.2025**
(21) Application number: 19838340.8
(22) Date of filing: 19.07.2019
(51) Int. Cl.: A61K 45/00, A61K 39/395, A61P 25/00, A61P 31/12, A61P 43/00, C07K 16/22

(54) **AGENT FOR TREATMENT OR PREVENTION OF HTLV-1-ASSOCIATED MYELOPATHY (HAM), AND HAM TREATMENT METHOD**
MITTEL ZUR BEHANDLUNG ODER VORBEUGUNG VON MIT HTLV-1-ASSOZIIERTER MYELOPATHIE (HAM) UND VERFAHREN ZUR BEHANDLUNG VON HAM
AGENT POUR LE TRAITEMENT OU LA PRÉVENTION DE LA MYÉLOPATHIE ASSOCIÉE AU HTLV-1 (HAM), ET MÉTHODE DE TRAITEMENT DE HAM

(30) Priority: 19.07.2018 JP 2018135925
(43) Date of publication of application: 26.05.2021
(73) Proprietor: The University of Tokyo, Bunkyo-ku, Tokyo 113-8654 (JP); St. Marianna University School of Medicine, Kawasaki-shi Kanagawa 216-8511 (JP)
(72) Inventor: UCHIMARU, Kaoru, Tokyo 113-8654 (JP); YAMAGISHI, Makoto, Tokyo 113-8654 (JP); ISHIZAKI, Izumi, Tokyo 113-8654 (JP); YAMANO, Yoshihisa, Kawasaki-shi, Kanagawa 216-8511 (JP)
(74) Representative: Hoffmann Eitle
(86) International application number: PCT/JP2019/028431
(87) International publication number: WO 2020/017629

(56) References cited:
- EP-A1- 3 290 441
- WO-A1-2005/087268
- WO-A1-2014/007303
- WO-A1-2014/007303
- WO-A1-2016/175236
- WO-A1-2016/175236
- WO-A1-2017/210278
- JP-A- 2006 516 594
- JP-A- 2013 119 531
- US-B2- 9 675 672
- YAMAUCHI JUNJI ET AL: "An update on human T-cell leukemia virus type I (HTLV-1)-associated myelopathy/tropical spastic paraparesis (HAM/TSP) focusing on clinical and laboratory biomarkers", PHARMACOLOGY & THERAPEUTICS, ELSEVIER, GB, vol. 218, 21 August 2020 (2020-08-21), XP086440949, ISSN: 0163-7258, [retrieved on 20200821], DOI: 10.1016/J.PHARMTHERA.2020.107669
- MOTHE ANDREA J. ET AL: "RGMa inhibition with human monoclonal antibodies promotes regeneration, plasticity and repair, and attenuates neuropathic pain after spinal cord injury", SCIENTIFIC REPORTS, vol. 7, no. 1, 1 December 2017 (2017-12-01), XP055834756, Retrieved from the Internet <URL:https://www.ncbi.nlm.nih.gov/pmc/articles/PMC5585220/pdf/41598_2017_Article_10987.pdf> DOI: 10.1038/s41598-017-10987-7

## Description

### Technical Field

The present invention relates to a therapeutic or prophylactic antibodies for HTLV-1-associated myelopathy (HAM). The present invention also relates to the use of said antibodies in a method for treating HAM.

### Background Art

HTLV-1 (human T-cell leukemia virus type 1) is a virus that infects T-cells and the like (mainly CD4-positive T-cells), which are kind of leukocytes in blood.

T-cells infected with HTLV-1 form chronic inflammation in the spinal cord and as a result, cause the disorder and degeneration of spinal cord neuronal cells, inducing spastic spinal cord paralysis. Spastic spinal cord paralysis caused by cells infected with HTLV-1 is called HTLV-1-associated myelopathy (also abbreviated to HAM).

Examples of the symptoms of HAM include symptoms ascribable to neuronal tissue damage such as paralysis in both legs, pain, dysuria, and obstinate constipation. As these symptoms progress, patients become confined to a wheelchair or bed. HAM is one of the diseases designated as a designated intractable disease in Japan. No effective method for treating HAM has yet to be established, and symptomatic therapy is mainly practiced.

As one of the treatment methods, a treatment method using an anti-CCR4 antibody has been proved to exert an effect of improving symptoms by decreasing the number of HTLV-1-infected cells and reducing spinal cord inflammation of HAM (Non-Patent Literature 1 and Patent Literature 5).

RGMa protein is a member of the RGM (repulsive guidance molecule) protein family involved in the axon guidance of retinal or hippocampal neuronal cells, neural tube closure and the like. The functions of RGM are not limited thereto, and this protein is known to have various functions.

For example, Patent Literature 1 discloses that: RGM is expressed in bone marrow-derived dendritic cells (BMDCs); and an RGM receptor is expressed in CD4⁺ T-cells and CD11b⁺ macrophages, and RGM binds to the RGM receptor, thereby enhancing the cell adhesion activity of the CD4⁺ T-cells and the CD11b⁺ macrophages. Patent Literature 1 also discloses that: an anti-RGM neutralizing antibody can reduce both the clinical symptoms and tissue lesions of multiple sclerosis mouse models; and antigen-specific and nonspecific T-cell activation was attenuated in spleen cells obtained from the mice.

Patent Literatures 2 and 3 each disclose a neutralizing monoclonal antibody against RGMa selectively inhibiting the binding of RGMa to an RGMa receptor neogenin and bone morphogenic proteins 2 and 4 (BMP-2 and BMP-4). The neutralizing monoclonal antibody is reportedly capable of promoting the nerve regeneration and regrowth of disrupted neuronal connection in the human central nervous system with damage and inflammation, specifically, neurodegenerative diseases such as multiple sclerosis, acute spinal cord injury, brain trauma, Huntington's chorea, Parkinson's disease, Alzheimer's disease, and the like.

Patent Literature 4 discloses a method for detecting and quantifying an RGMa fragment for the purpose of diagnosing neurodegenerative diseases such as traumatic brain injury and ischemic stroke because RGMa is localized in the central nervous system myelin, new lesions and mature scar tissues of humans having traumatic brain injury or ischemic stroke. Patent Literature 4 also lists multiple sclerosis, Parkinson's disease, Alzheimer's disease, Tay-Sachs disease, Niemann-Pick disease, Gaucher's disease, Hurler syndrome, Huntington's disease, amyotrophic lateral sclerosis, idiopathic inflammatory demyelinating disease, vitamin B12 deficiency, central pontine myelinolysis, tabes dorsalis, transverse myelitis, Devic disease, progressive multifocal leukoencephalopathy, optic neuritis, spinal cord injury, traumatic brain injury, stroke, glaucoma, diabetic retinopathy, age-related macular degeneration, and leukodystrophy as target neurodegenerative diseases and disorders for the detection of the RGMa fragment. Patent literature 6 discloses a treatment for HTLV-1 associated myelopathy (HAM) using an CC-chemokine receptor 4 (CCR4) antibody.

### Citation List

### Patent Literature

Patent Literature 1: International Publication No. WO 2011/071059
Patent Literature 2: Japanese Patent Laid-Open No. 2014-138599
Patent Literature 3: Japanese Patent Laid-Open No. 2016-175897
Patent Literature 4: Japanese Translation of PCT International Application Publication No. 2017-526930
Patent Literature 5: Japanese Patent Laid-Open No. 2010-100578 Patent Literature 6: International Pubblication No. WO 2014/007303

### Non-Patent Literature

Non-Patent Literature 1: N Engl J Med, 2018, 378, 529-538

### Summary of Invention

### Technical Problem

Methods using an anti-CCR4 antibody as disclosed in Non-Patent Literature 1 and Patent Literature 5 can improve the symptoms of HAM. However, the anti-CCR4 antibody has a limited effect on HAM patients having advanced neuronal destruction. Hence, there is a demand for a better method that can treat HAM.

The present invention has been made in light of these problems. An object of the present invention is to provide a therapeutic agent and a treatment method that can treat HAM.

### Solution to Problem

The present inventors have conducted diligent studies to attain the object and consequently completed the present invention by finding that antibodies inhibiting RGMa are effective for the treatment of HAM.

### Advantageous Effects of Invention

According to the present invention, HAM, an intractable disease, can be treated.

### Brief Description of Drawings

[Figure 1] Figure 1 is a diagram showing groups, of CD4-positive T-cells, P, D and N classified according to CADM1 and CD7 serving as indexes for HTLV-1-infected cells.

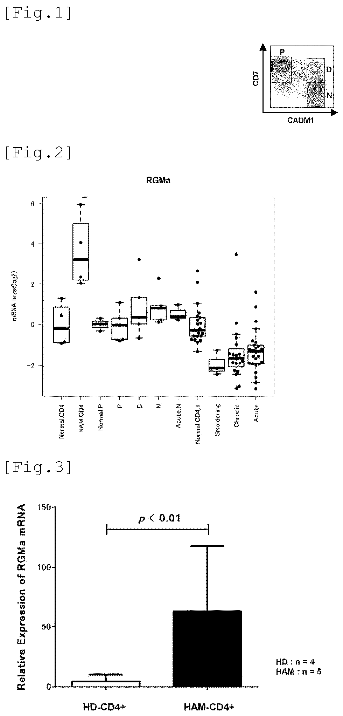

[Figure 2] Figure 2 is a diagram showing the expression level of RGMa gene in normal T-cells (Normal.CD4), HAM patient-derived CD4-positive T-cells (HAM.CD4), healthy person-derived CD4-positive/CADM1-negative/CD7-positive T-cells (Normal.P), HTLV-1-infected person-derived CD4-positive/CADM1-negative/CD7-positive T-cells (P group), HTLV-1-infected person-derived CD4-positive/CADM1-positive/CD7-positive T-cells (D group), HTLV-1-infected person-derived CD4-positive/CADM1-positive/CD7-negative T-cells (N group), acute ATL patient-derived CD4-positive/CADM1-positive/CD7-negative T-cells (Acute.N), healthy person-derived CD4-positive T-cells (Normal.CD4.1), smoldering ATL patient-derived PBMCs (Smoldering), chronic ATL patient-derived PBMCs (Chronic), and acute ATL patient-derived PBMCs (Acute).
[Figure 3] Figure 3 is a diagram showing the expression level of RGMa in HAM patient-derived CD4-positive T-cells and healthy person-derived CD4-positive T-cells.
[Figure 4] Figure 4 is a diagram showing results of analyzing the expression level of RGMa among the cell species of HAM patient-derived PBMCs.
[Figure 5] Figure 5 is a diagram showing change in the expression of RGMa associated with the expression of HTLV-1 virus during the culture of HAM patient PBMCs.
[Figure 6] Figure 6 is a diagram showing an H3K27me3 level at or around -2,916 bp upstream from the transcription start point of RGMa gene.
[Figure 7] Figure 7 is a diagram showing the mRNA level of RGMa gene in a human CD4-positive T-cell leukemia cell line Jurkat transfected with a lentivirus vector having an insert of cDNA encoding HTLV-1 Tax.
[Figure 8] Figure 8 is a diagram showing results of analyzing the protein expression of Tax and RGMa in a cell line JPX-9 having expression of HTLV-1-tax induced.
[Figure 9] Figure 9 is a diagram showing results about the effect of an anti-RGMa antibody on spontaneous proliferative activity as to the effect of the anti-RGMa antibody on HAM patient PBMCs.
[Figure 10] Figure 10 is a diagram showing results about the effect of the anti-RGMa antibody on change in the amount of HTLV-1 provirus as to the effect of the anti-RGMa antibody on HAM patient PBMCs.
[Figure 11] Figure 11 is a diagram showing results about the effect of the anti-RGMa antibody on CXCL10 production as to the effect of the anti-RGMa antibody on HAM patient PBMCs.
[Figure 12] Figure 12 is a diagram showing results about the effect of the anti-RGMa antibody on the cytokine production of HAM patient PBMCs as to the effect of the anti-RGMa antibody on HAM patient PBMCs.
[Figure 13] Figure 13 is a diagram showing that HAM-PBMCs induced the apoptosis of a neuronal cell line.

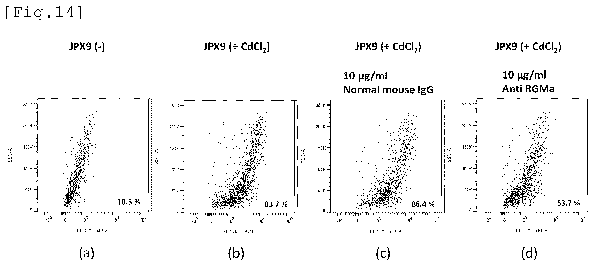

[Figure 14] Figure 14 is a diagram showing results about the suppressive effect of the anti-RGMa antibody on the induction of apoptosis of a neuronal cell line by a cell line having HTLV-1-tax induced. Figure 14(a) is a FACS plot when NB-1 cells and JPX-9 cells (unstimulated) were cocultured. Figure 14(b) is a FACS plot when NB-1 cells and CdCl₂-stimulated JPX-9 cells (HTLV-1-tax-expressing cells) were cocultured. Figure 14(c) is a FACS plot when a control antibody was added under the conditions of Figure 14(b). Figure 14(d) is a FACS plot when the anti-RGMa antibody was added under the conditions of Figure 14 (b) .

### Description of Embodiments

Hereinafter, embodiments of the present invention will be described in detail. However, the present invention is not limited by the present embodiment given below, but as set in the claims. Any references in the description to methods of treatment refer to the compounds, pharmaceutical compositions and medicaments of the present invention for use in a method for treatment of the human (or animal) body by therapy (or for diagnosis)

The present invention provides a therapeutic or prophylactic agent for HTLV-1-associated myelopathy (HAM), comprising an RGMa-inhibiting substance. The RGMa-inhibiting substance may be a substance that inhibits the activity of RGMa by acting on RGMa itself or may be a substance that suppresses the expression of RGMa.

Examples of the RGMa-inhibiting substance include compounds having activity of inhibiting RGMa and antibodies recognizing RGMa.

Examples of the RGMa-inhibiting substance also include substances suppressing the expression of RGMa such as siRNA (short interfering RNA), shRNA (short hairpin RNA), antisense oligonucleotides and the like, against a gene expressing RGMa.

Examples of the RGMa gene include, but are not limited to, human RGMa gene consisting of the nucleotide sequence represented by SEQ ID NO: 1, and RGMa gene consisting of the nucleotide sequence represented by SEQ ID NO: 2. Information on the nucleotide sequence of the RGMa gene derived from each of various organisms can be obtained from a database such as GenBank and the like.

The siRNA is double-stranded RNA that can suppress the expression of the target RGMa gene. The length of the nucleotide sequence (base length) of the siRNA is not limited but it is preferably less than approximately 30 bases, more preferably approximately 19 to 27 bases, further preferably approximately 21 to 25 bases.

The shRNA refers to a molecule of approximately 20 base pairs or more composed of a short hairpin structure that has an intramolecular double-stranded structure formed by containing a palindrome nucleotide sequence in a part of single-stranded RNA, and also has a 3'-terminal overhang. The shRNA, after being introduced into a cell, is to be decomposed into a length of approximately 20 bases in the cell and can suppress the expression of the target RGMa gene, as in siRNA.

The siRNA and the shRNA can be chemically synthesized in an artificial manner. For the siRNA and the shRNA, antisense and sense strands of the RNA can be synthesized *in vitro* from template DNA using, for example, T7RNA polymerase and T7 promoter.

The antisense oligonucleotide can be any nucleotide that is complementary to or hybridizes to a nucleotide sequence of less than approximately 30 consecutive bases in the DNA sequence of the RGMa gene, and may be any of DNA and RNA. The antisense oligonucleotide may be modified without hindering its functions. The antisense oligonucleotide can be synthesized by a common method and can be readily synthesized using, for example, a commercially available DNA synthesis apparatus.

The RGMa-inhibiting substance contained in the therapeutic or prophylactic agent for HAM of the present invention is preferably an antibody recognizing RGMa. Hereinafter, the antibody recognizing RGMa is also referred to as an anti-RGMa antibody. The anti-RGMa antibody according to the present invention can be any antibody that inhibits the activity of RGMa by binding the RGMa. Examples thereof include antibodies that binds to RGMa resulting in preventing the RGMa from binding to an RGMa receptor.

The anti-RGMa antibody according to the present invention may be a monoclonal antibody or a polyclonal antibody. The antibody according to the present invention may be any of isotypes of IgG, IgM, IgA, IgD and IgE.

The anti-RGMa antibody according to the present invention may be, for example, a mouse antibody, a human-type CDR-grafted antibody, a human-type chimeric antibody, a humanized antibody, or a complete human antibody, or may be a small antibody. One of these antibodies may be used singly, or two or more thereof may be used in combination.

The human-type CDR-grafted antibody is an antibody obtained by replacing CDRs of a non-human animal antibody with CDRs of a human antibody. The human-type chimeric antibody is an antibody composed of variable regions derived from a non-human animal antibody and constant regions derived from a human antibody. The humanized antibody refers to an antibody obtained by incorporating a moiety derived from a human antibody into a non-human animal antibody with a highly safe partial region remaining therein, and conceptually includes the human-type chimeric antibody and the human-type CDR-grafted antibody.

In the present specification, the "small antibody" refers to an antibody fragment or an antibody fragment bound with an arbitrary molecule that recognizes the same epitope as that for the original antibody. Specific examples thereof include, but are not limited to: Fab composed of VL, VH, CL and CH1 regions; F(ab')2 in which two Fab molecules are linked through a disulfide bond at their hinge regions; Fv composed of VL and VH; a singlechain antibody scFv in which VL and VH are linked via an artificial polypeptide linker; and sdFv, Diabody, and sc(Fv)2.

The anti-RGMa antibody used in the present invention can be prepared by a method known in the art using RGMa or its fragment as an immunogen.

Whether the obtained antibody is an anti-RGMa antibody can be confirmed by using RGMa activity as an index.

Examples of the RGMa include human RGMa comprising the amino acid sequence represented by SEQ ID NO: 3, and RGMa comprising the amino acid sequence represented by SEQ ID NO: 4. RGMa derived from each of various organisms can be used as the immunogen. The amino acid sequence of RGMa can be obtained from a database known in the art, such as Protein Data Bank and the like.

When the anti-RGMa antibody used in the present invention is a polyclonal antibody, this polyclonal antibody can be prepared, for example, as follows: RGMa or its fragment as an antigen is dissolved in phosphatebuffered saline (also referred to as PBS) and mixed, if necessary, with an appropriate amount of a usual adjuvant such as Freund's complete adjuvant. This mixture is used as an immunogen to immunize a mammal such as a mouse, a rat, a rabbit, a goat, a horse and the like. Examples of the immunization method include, but are not limited to, a method of performing subcutaneous injection or intraperitoneal injection once or two or more times at an appropriate interval. Subsequently, blood is collected from the immunized animal according to a common method, and serum is separated. A polyclonal antibody fragment can be purified to obtain the polyclonal antibody.

When the anti-RGMa antibody used in the present invention is a monoclonal antibody, this monoclonal antibody can be obtained by fusing immune cells, for example, spleen cells, obtained from the immunized mammal with myeloma cells to obtain hybridomas, and collecting an antibody from cultures of the hybridomas. Also, the monoclonal antibody may be produced as a recombinant monoclonal antibody by use of a gene recombination technique which involves cloning an antibody gene from the hybridoma, incorporating the antibody gene into an appropriate vector, and transfecting host cells therewith. Alternatively, the monoclonal antibody may be prepared by a phage display method.

An antibody disclosed in, for example, Yamashita, T., Mueller, B.K. & Hata, K. Neogenin and repulsive guidance molecule signaling in the central nervous system. Curr. Opin. Neurobiol. 17, 29-34 (2007); Japanese Patent Laid-Open No. 2014-138599; Japanese Patent Laid-Open No. 2016-175897; Japanese Translation of PCT International Application Publication No. 2017-526930; International Publication No. WO 2016/175236; Japanese Translation of PCT International Application Publication No. 2015-508061 or the like can be used as the anti-RGMa antibody used in the present invention.

The anti-RGMa antibody used in the present invention may be obtained as a commercially available product manufactured by, for example, Immuno-Biological Laboratories Co., Ltd. (IBL), R&D Systems, Inc. or the like.

The anti-RGMa antibody preferably comprises, as an antigen-binding domain, at least one CDR comprising an amino acid sequence selected from the group consisting of:
GTTPDY (SEQ ID NO: 7);
FQATHDPLT (SEQ ID NO: 10);
ARRNEYYGSSFFDY (SEQ ID NO: 13);
LQGYIPPRT (SEQ ID NO: 16); and
a modified CDR amino acid sequence having at least 50% sequence identity to one of these sequences. The sequence identity described above is preferably 80% or higher, more preferably 90% or higher. In the present specification, each amino acid is represented by a conventional single-letter code or three-letter code.

The complementarity-determining region (CDR) refers to a region that forms an antigen-binding site, of a variable region of an immunoglobulin molecule. This region, also called hypervariable region, is a moiety having particularly large variability of an amino acid sequence in each immunoglobulin molecule. A light chain and a heavy chain each have three CDRs (CDR-L1, CDR-L2, and CDR-L3, and CDR-H1, CDR-H2, and CDR-H3). In the present application, CDRs of an immunoglobulin molecule are determined according to the numbering system of Kabat (Kabat et al., 1987, Sequences of Proteins of Immunological Interest, US Department of Health and Human Services, NIH, USA).

In the antibody defined by the amino acid sequences of a light chain and a heavy chain according to the present invention, the amino acid sequences of CDRs are fixed to predetermined sequences, and a region other than the CDRs may be altered by a mutation or the like. When the region other than CDRs has a mutation or the like, the homology is preferably 90% or higher.

The anti-RGMa antibody preferably further comprises at least one CDR comprising an amino acid sequence selected from the group consisting of SEQ ID NOs: 5, 6, 8, 9, 11, 12, 14 and 15 and a modified CDR amino acid sequence having at least 50% sequence identity to one of these sequences. The sequence identity is preferably 80% or higher, more preferably 90% or higher.

The anti-RGMa antibody more preferably comprises at least three CDRs selected from variable domain CDR sets shown in Table 1, or variable domain sets in which at least one of the three CDRs has a modified CDR amino acid sequence having at least 50%, preferably 80%, more preferably 90% sequence identity to the parent sequence. The anti-RGMa antibody further preferably comprises at least two of the variable domain CDR sets shown in Table 1. The at least two variable domain CDR sets are preferably a combination of the VH5F9 set and the VL5F9 set, or a combination of the VH8D1 set and the VL8D1 set.

**[Table 1]**

| VH5F9 set | | |
|---|---|---|
| VH5F9 CDR-H1 | Residues 31-35 of SEQ ID NO: 17 | SEQ ID NO: 5 |
| VH5F9 CDR-H2 | Residues 50-66 of SEQ ID NO: 17 | SEQ **ID** NO: 6 |
| VH5F9 CDR-H3 | Residues 99-104 of SEQ ID NO: 17 | SEQ ID NO: 7 |

| VL5F9 set | | |
|---|---|---|
| VL5F9 CDR-L1 | Residues 24-39 of SEQ ID NO: 18 | SEQ ID NO: 8 |
| VL5F9 CDR-L2 | Residues 55-61 of SEQ ID NO: 18 | SEQ **ID** NO: 9 |
| VL5F9 CDR-L3 | Residues 94-102 of SEQ ID NO: 18 | SEQ ID NO: 10 |

| VH8D1 set | | |
|---|---|---|
| VH8D1 CDR-H1 | Residues 31-35 of SEQ ID NO: 19 | SEQ ID NO: 11 |
| VH8D1 CDR-H2 | Residues 50-66 of SEQ ID NO: 19 | SEQ ID NO: 12 |
| VH8D1 CDR-H3 | Residues 97-110 of SEQ ID NO: 19 | SEQ ID NO: 13 |

| VL8D1 set | | |
|---|---|---|
| VL8D1 CDR-L1 | Residues 24-34 of SEQ ID NO: 20 | SEQ ID NO: 14 |
| VL8D1 CDR-L2 | Residues 50-56 of SEQ ID NO: 20 | SEQ ID NO: 15 |
| VL8D1 CDR-L3 | Residues 89-97 of SEQ ID NO: 20 | SEQ ID NO: 16 |

The anti-RGMa antibody may comprise a framework region. Examples of an amino acid sequence contained in the framework region include SEQ ID NOs: 22, 23, 24, 25, 26, 27, 28, 29, 30, 31, 32, 33, 34, 35, 36, 37, 38, 39 and 40. One of these amino acid sequences may be used singly, or two or more thereof may be combined.

The anti-RGMa antibody preferably comprises at least one heavy chain variable domain selected from SEQ ID NOs: 41, 42, 43, 44, 45, 46, 47, 48 and 49; and/or at least one light chain variable domain selected from SEQ ID NOs: 50, 51 and 52, as a heavy chain variable domain and a light chain variable domain.

The binding site of the anti-RGMa antibody in binding to RGMa is not limited as long as the site causes the inhibition of RGMa. For example, when the RGMa is human RGMa, the anti-RGMa antibody preferably binds to one or more peptides having the amino acid sequence represented by
EEVVNAVEDWDSQG (SEQ ID NO: 53),
NQQIDFQAFHTNAE (SEQ ID NO: 54),
PTAPETFPYET (SEQ ID NO: 55),
KLPVEDLYYQA (SEQ ID NO: 56), or
LYERTRDLPGRAAAGL (SEQ ID NO: 57).

The anti-RGMa antibody more preferably binds to a peptide having the amino acid sequence represented by SEQ ID NO: 53 and/or SEQ ID NO: 54, and more preferably binds to a peptide having the amino acid sequence represented by SEQ ID NO: 53 and/or SEQ ID NO: 54, and a peptide having the amino acid sequence represented by SEQ ID NO: 55 and/or SEQ ID NO: 56.

The anti-RGMa antibody is preferably an antibody binding to any position of 250th and the subsequent positions in the amino acid sequence of human RGMa.

The anti-RGMa antibody more preferably binds to a peptide having the amino acid sequences represented by SEQ ID NO: 53 and SEQ ID NO: 54, and SEQ ID NO: 55 or SEQ ID NO: 56.

The anti-RGMa antibody may be, for example, a polyclonal antibody and a monoclonal antibody obtained by using RGMa protein or its partial fragment (e.g., a fragment comprising one or more of SEQ ID NOs: 53 to 57) as an antigen and immunizing a mammal such as a mouse with the antigen, a chimeric antibody and a humanized antibody produced by use of a gene recombination technique, a human antibody produced by using a human antibody-producing transgenic animal, and the like.

In the present invention, the anti-RGMa antibody, when administered as a medicament to a human, is desirably a humanized antibody or a human antibody from the viewpoint of adverse reaction.

The anti-RGMa antibody may be a partial fragment of a polyclonal antibody and/or a monoclonal antibody recognizing RGMa protein or its partial fragment (e.g., a fragment comprising one or more of SEQ ID NOs: 53 to 57) as an antigen, or may be a small antibody.

The anti-RGMa antibody may be an isolated anti-RGMa antibody or an antigen-binding fragment thereof as well as that shown in Table 1, wherein the amino acid sequences, of the anti-RGMa antibody, of light chain complementarity-determining region 1 (LCDR1), light chain complementarity-determining region 2 (LCDR2), light chain complementarity-determining region 3 (LCDR3), heavy chain complementarity-determining region 1 (HCDR1), heavy chain complementarity-determining region 2 (HCDR2) and heavy chain complementarity-determining region 3 (HCDR3) comprise
LCDR1: RASQDISSYLN (SEQ ID NO: 58),
LCDR2: YTSRLHS (SEQ ID NO: 59),
LCDR3: QQLNTLP (SEQ ID NO: 60),
HCDR1: DAWMD (SEQ ID NO: 61),
HCDR2: EIRSKANNHATYYAESVKG (SEQ ID NO: 62) and
HCDR3: RDGAY (SEQ ID NO: 63), respectively,
   or
LCDR1: RSSQSLVHSNGNTYLH (SEQ ID NO: 64),
LCDR2: KVSNRFS (SEQ ID NO: 65),
LCDR3: SQSTHVP (SEQ ID NO: 66),
HCDR1: TSYYWN (SEQ ID NO: 67),
HCDR2: YISYDGTNNYNPSLKN (SEQ ID NO: 68) and
HCDR3: SFG, respectively.

In each of the CDR sequences, one or several amino acids may be substituted, deleted, and/or added. For example, one or two amino acids may be substituted, deleted, and/or added.

Examples of the anti-RGMa antibody include an antibody having the amino acid sequence of SEQ ID NO: 73 in a light chain and the amino acid sequence of SEQ ID NO: 74 in a heavy chain. In each of the amino acid sequences represented by these SEQ ID NOs, one or several (1 to 20, 1 to 10 or 1 to 5) amino acids may be substituted, deleted, added or inserted. Such substitution, deletion or addition may be introduced in CDR but is preferably introduced in a region other than CDR.

A mouse/human chimeric antibody having a human-derived constant region may be used. Examples of the mouse/human chimeric antibody include an antibody having the amino acid sequence of SEQ ID NO: 77 (variable region: positions 1 to 107) in a light chain and the amino acid sequence of SEQ ID NO: 78 (variable region: positions 1 to 116) in a heavy chain. In each of the amino acid sequences represented by these SEQ ID NOs, one or several (1 to 20, 1 to 10 or 1 to 5) amino acids may be substituted, deleted, added or inserted. Such substitution, deletion or addition may be introduced in CDR but is preferably introduced in a region other than CDR.

A humanized antibody having a human-derived region other than CDRs may be used. Examples of the humanized antibody include an antibody having the amino acid sequence of any of SEQ ID NOs: 70 to 87 (variable region: up to 116 residues from N-terminal) in a heavy chain and the amino acid sequence of any of SEQ ID NOs: 88 to 94 (variable region: 1 to 107 residues from N-terminal) in a light chain. In each of the amino acid sequences represented by these SEQ ID NOs, one or several (1 to 20, 1 to 10 or 1 to 5) amino acids may be substituted, deleted, added or inserted. Such substitution, deletion or addition may be introduced in CDR but is preferably introduced in a region other than CDR.

The amino acid sequence of the heavy chain and the amino acid sequence of the light chain can be arbitrarily combined. An antibody having the amino acid sequence of SEQ ID NO: 84 in a heavy chain and the amino acid sequence of SEQ ID NO: 88 in a light chain is preferred. In the amino acid sequence of SEQ ID NO: 84, an amino acid sequence corresponding to a heavy chain variable region is represented by SEQ ID NO: 95, and an amino acid sequence corresponding to a light chain variable region is represented by SEQ ID NO: 96.

The anti-RGMa antibody is preferably an isolated anti-RGMa antibody or an antigen-binding fragment thereof, wherein
a heavy chain variable region (VH) comprises EVQLVESGGGLVQPGRSLRLSCTASGFTFSDAWMDWVRQAPGKGLEWVAEIRSKANN HATYYAESVKGRFTISRDDSKSIVYLQMNSLRTEDTALYYCTRRDGAYWGKGTTVTV SS (SEQ ID NO: 95) or an amino acid sequence having at least 90% identity to this amino acid sequence, and
a light chain variable region (VL) comprises DIQMTQSPSSVSASVGDRVTITCRASQDISSYLNWYQQKPGKAPKLLIYYTSRLHSG VPSRFSGSGSGTDFTLTISSLQPEDFASYFCQQLNTLPWTFGGGTKVEME (SEQ ID NO: 96) or an amino acid sequence having at least 90% identity to this amino acid sequence.

Examples of the anti-RGMa antibody include an antibody having the amino acid sequence of SEQ ID NO: 75 in a light chain and the amino acid sequence of SEQ ID NO: 76 in a heavy chain. In each of the amino acid sequences represented by these SEQ ID NOs, one or several (1 to 20, 1 to 10 or 1 to 5) amino acids may be substituted, deleted, added or inserted. Such substitution, deletion or addition may be introduced in CDR but is preferably introduced in a region other than CDR.

The anti-RGMa antibody may be a mouse/human chimeric antibody having a human-derived constant region or may be a humanized antibody having a human-derived region other than CDRs.

The anti-RGMa antibody may be an isolated anti-RGMa antibody or an antigen-binding fragment thereof selected from the following (a1) to (h1):
(a1) an anti-RGMa antibody comprising a light chain variable region comprising LCDR1 comprising the amino acid sequence represented by SEQ ID NO: 97, LCDR2 comprising the amino acid sequence represented by SEQ ID NO: 98 and LCDR3 comprising the amino acid sequence represented by SEQ ID NO: 99, and a heavy chain variable region comprising HCDR1 comprising the amino acid sequence represented by SEQ ID NO: 100, HCDR2 comprising the amino acid sequence represented by SEQ ID NO: 101 and HCDR3 comprising the amino acid sequence represented by SEQ ID NO: 102, or an antigen-binding fragment thereof,
(b1) an anti-RGMa antibody comprising a light chain variable region comprising LCDR1 comprising the amino acid sequence represented by SEQ ID NO: 97, LCDR2 comprising the amino acid sequence represented by SEQ ID NO: 98 and LCDR3 comprising the amino acid sequence represented by SEQ ID NO: 103, and a heavy chain variable region comprising HCDR1 comprising the amino acid sequence represented by SEQ ID NO: 100, HCDR2 comprising the amino acid sequence represented by SEQ ID NO: 101 and HCDR3 comprising the amino acid sequence represented by SEQ ID NO: 102, or an antigen-binding fragment thereof,
(c1) an anti-RGMa antibody comprising a light chain variable region comprising LCDR1 comprising the amino acid sequence represented by SEQ ID NO: 97, LCDR2 comprising the amino acid sequence represented by SEQ ID NO: 98 and LCDR3 comprising the amino acid sequence represented by SEQ ID NO: 104, and a heavy chain variable region comprising HCDR1 comprising the amino acid sequence represented by SEQ ID NO: 100, HCDR2 comprising the amino acid sequence represented by SEQ ID NO: 101 and HCDR3 comprising the amino acid sequence represented by SEQ ID NO: 102, or an antigen-binding fragment thereof,
(d1) an anti-RGMa antibody comprising a light chain variable region comprising LCDR1 comprising the amino acid sequence represented by SEQ ID NO: 97, LCDR2 comprising the amino acid sequence represented by SEQ ID NO: 98 and LCDR3 comprising the amino acid sequence represented by SEQ ID NO: 105, and a heavy chain variable region comprising HCDR1 comprising the amino acid sequence represented by SEQ ID NO: 100, HCDR2 comprising the amino acid sequence represented by SEQ ID NO: 101 and HCDR3 comprising the amino acid sequence represented by SEQ ID NO: 102, or an antigen-binding fragment thereof,
(e1) an anti-RGMa antibody comprising a light chain variable region comprising LCDR1 comprising the amino acid sequence represented by SEQ ID NO: 97, LCDR2 comprising the amino acid sequence represented by SEQ ID NO: 98 and LCDR3 comprising the amino acid sequence represented by SEQ ID NO: 106, and a heavy chain variable region comprising HCDR1 comprising the amino acid sequence represented by SEQ ID NO: 100, HCDR2 comprising the amino acid sequence represented by SEQ ID NO: 101 and HCDR3 comprising the amino acid sequence represented by SEQ ID NO: 102, or an antigen-binding fragment thereof,
(f1) an anti-RGMa antibody comprising a light chain variable region comprising LCDR1 comprising the amino acid sequence represented by SEQ ID NO: 97, LCDR2 comprising the amino acid sequence represented by SEQ ID NO: 98 and LCDR3 comprising the amino acid sequence represented by SEQ ID NO: 107, and a heavy chain variable region comprising HCDR1 comprising the amino acid sequence represented by SEQ ID NO: 100, HCDR2 comprising the amino acid sequence represented by SEQ ID NO: 101 and HCDR3 comprising the amino acid sequence represented by SEQ ID NO: 102, or an antigen-binding fragment thereof,
(g1) an anti-RGMa antibody comprising a light chain variable region comprising LCDR1 comprising the amino acid sequence represented by SEQ ID NO: 97, LCDR2 comprising the amino acid sequence represented by SEQ ID NO: 98 and LCDR3 comprising the amino acid sequence represented by SEQ ID NO: 108, and a heavy chain variable region comprising HCDR1 comprising the amino acid sequence represented by SEQ ID NO: 100, HCDR2 comprising the amino acid sequence represented by SEQ ID NO: 101 and HCDR3 comprising the amino acid sequence represented by SEQ ID NO: 102, or an antigen-binding fragment thereof, and
(h1) an anti-RGMa antibody comprising a light chain variable region comprising LCDR1 comprising the amino acid sequence represented by SEQ ID NO: 97, LCDR2 comprising the amino acid sequence represented by SEQ ID NO: 98 and LCDR3 comprising the amino acid sequence represented by SEQ ID NO: 109, and a heavy chain variable region comprising HCDR1 comprising the amino acid sequence represented by SEQ ID NO: 100, HCDR2 comprising the amino acid sequence represented by SEQ ID NO: 101 and HCDR3 comprising the amino acid sequence represented by SEQ ID NO: 102, or an antigen-binding fragment thereof.

The anti-RGMa antibody is preferably an antibody that has the amino acid sequences mentioned above and that is a humanized antibody, and also preferably has a constant region of human IgG.

HAM can be treated or prevented with the RGMa-inhibiting substance according to the present invention.

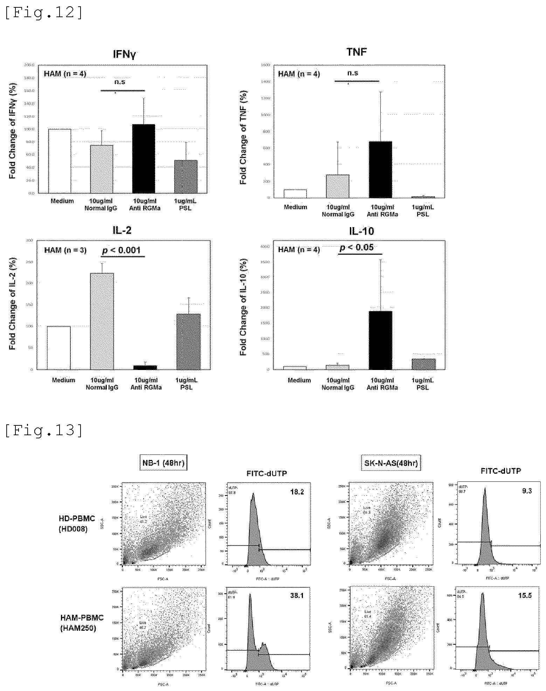

As shown in Examples mentioned later and Figure 13, neuronal cell death, i.e., injury and degeneration of spinal cord tissues, is induced by HAM patient-derived cells in HAM patients.

As a result of studies of the present inventors, as shown in Examples mentioned later, RGMa was markedly expressed in CD4-positive T-cells which are main HTLV-1-infected cells in HAM patients, demonstrating that the expression of RGMa is involved in HAM. Results of tests to inhibit RGMa using the anti-RGMa antibody demonstrated that RGMa is involved in the production of CXCL10, IL-2, and IL-10.

CXCL10 is a protein that is produced from HAM-pathogenic HTLV-1-infected T-cells in response to IFN-y. This protein is known to induce the pathological condition of HAM (Brain 2013) and also known to correlate strongly with the progression rate of symptoms of HAM (PLoS Negl Trop Dis 2013). The administration of the anti-RGMa antibody on cells of HAM patients suppressed the production of this CXCL10. IL-10, which is one of cytokines, works to suppress inflammation. The administration of the anti-RGMa antibody on cells of HAM patients markedly increased the production of IL-10.

For bringing about a true therapeutic effect on HAM, it is required to suppress damage on neuronal cells. HTLV-1-infected cells of HAM patients have a high expression level of HTLV-1-tax (Blood 2002), and HTLV-1-tax has been shown to be important for the formation of the pathological condition (J Clin Invest 2014). In the present invention, it was shown that HTLV-1-tax induces the expression of RGMa and HAM patient-derived cells having a high RGMa expression level induce neuronal cell death, and importantly, it was shown that the RGMa-inhibiting substance suppresses neuronal cell death ascribable to HTLV-1-tax-expressing cells.

As described above, the RGMa-inhibiting substance can prevent the induction of the inflammatory pathological condition of HAM and suppress inflammatory reaction ascribable to HAM patient cells. Therefore, HAM can be treated or prevented therewith. Furthermore, the RGMa-inhibiting substance can not only suppress inflammatory reaction unique to HAM but also suppress neuronal cell death ascribable to HTLV-1-tax-expressing cells which are pathogenic cells of HAM patients. Therefore, HAM can be treated or prevented therewith.

As mentioned above, the RGMa-inhibiting substance can treat or prevent HAM. Thus, the present invention provides an RGMa-inhibiting substance for use in the treatment of HAM; a pharmaceutical composition for use in the treatment of HAM; use of an RGMa-inhibiting substance for treating HAM; use of an RGMa-inhibiting substance in the production of a medicament for the treatment of HAM; an RGMa-inhibiting substance for use in the production of a medicament for the treatment of HAM; and a method for treating or preventing HAM, comprising administering an effective amount of an RGMa-inhibiting substance to a subject in need thereof.

The therapeutic or prophylactic agent for HAM of the present invention comprises the RGMa-inhibiting substance and may be formulated by appropriately further blending a pharmaceutically acceptable carrier and/or additive therewith.

Examples of the form of formulation include: oral agents such as tablets, coated tablets, pills, powders, granules, capsules, solutions, suspension, emulsions and the like; and parenteral agents such as injections, transfusions, suppositories, ointments, patches and the like.

The blending ratio of the carrier or the additive can be appropriately set on the basis of a range usually adopted in the field of medicaments.

Examples of the carrier include, but are not limited to, water, saline, other aqueous solvents, and aqueous or oil bases. Examples of the additive include, but are not limited to, excipients, binders, pH adjusters, disintegrants, absorption promoters, lubricants, colorants, corrigents, and flavors.

When the RGMa-inhibiting substance according to the present invention is an antibody recognizing RGMa, this antibody is preferably administered as an injection or a transfusion formulated together with a pharmaceutically acceptable carrier through a parenteral administration route, for example, intravenously, intramuscularly, intradermally, intraperitoneally, subcutaneously or locally.

The injection or the transfusion comprising the anti-RGMa antibody can be used as a solution, a suspension or an emulsion. For example, distilled water for injection, saline, a glucose solution, an isotonic solution (e.g., sodium chloride, potassium chloride, glycerin, mannitol, sorbitol, boric acid, borax, and propylene glycol solutions) or the like can be used as a solvent therefor. One of these solvents may be used singly, or two or more thereof may be used in combination.

The injection or the transfusion may further contain an additive such as a stabilizer, a solubilizer, a suspending agent, an emulsifying agent, a soothing agent, a buffer, a preservative, an antiseptic, or a pH adjuster.

For example, albumin, globulin, gelatin, mannitol, glucose, dextran, ethylene glycol, propylene glycol, ascorbic acid, sodium bisulfite, sodium thiosulfate, sodium EDTA, sodium citrate, dibutylhydroxytoluene or the like can be used as the stabilizer.

For example, an alcohol (e.g., ethanol), a polyalcohol (e.g., propylene glycol and polyethylene glycol), a nonionic surfactant (e.g., Polysorbate 80(R) and HCO-50) or the like can be used as the solubilizer.

For example, glycerin monostearate, aluminum monostearate, methylcellulose, carboxymethylcellulose, hydroxymethylcellulose, sodium lauryl sulfate or the like can be used as the suspending agent.

For example, gum arabic, sodium alginate, tragacanth or the like can be used as the emulsifying agent.

For example, benzyl alcohol, chlorobutanol, sorbitol or the like can be used as the soothing agent.

For example, a phosphate buffer solution, an acetate buffer solution, a borate buffer solution, a carbonate buffer solution, a citrate buffer solution, a Tris buffer solution or the like can be used as the buffer.

For example, methyl p-hydroxybenzoate, ethyl p-hydroxybenzoate, propyl p-hydroxybenzoate, butyl p-hydroxybenzoate, chlorobutanol, benzyl alcohol, benzalkonium chloride, sodium dehydroacetate, sodium edetate, boric acid, borax or the like can be used as the preservative.

For example, benzalkonium chloride, p-hydroxybenzoic acid, chlorobutanol or the like can be used as the antiseptic.

For example, hydrochloric acid, sodium hydroxide, phosphoric acid, acetic acid or the like can be used as the pH adjuster.

When the RGMa-inhibiting substance according to the present invention is a substance suppressing the expression of RGMa, such as siRNA (short interfering RNA), shRNA (short hairpin RNA) or an antisense oligonucleotide against a gene expressing RGMa, this substance can be administered in the form of a non-virus vector or a virus vector.

When the RGMa-inhibiting substance is in the form of a non-virus vector, examples of the administration method can include methods of transferring nucleic acid molecules using liposomes (liposome method, HVJ-liposome method, cationic liposome method, lipofection method, lipofectamine method or the like.), microinjection methods, and methods of introducing nucleic acid molecules together with carriers (metal particles) into cells using gene guns.

In the case of administering siRNA or shRNA to an organism using a virus vector, a virus vector such as recombinant adenovirus or retrovirus can be used. By introducing DNA expressing siRNA or shRNA to a DNA virus or an RNA virus such as detoxified retrovirus, adenovirus, adeno-associated virus, herpesvirus, vaccinia virus, poxvirus, poliovirus, Sindbis virus, hemagglutinating virus of Japan, SV40 or the like, and infecting cells or tissues with this recombinant virus, the gene can be transferred into the cells or the tissues.

The preparation thus obtained can be administered in an effective amount to, for example, a human or any of other mammals such as a rat, a mouse, a rabbit, sheep, a pig, a horse, a cat, a dog, a monkey and the like to prevent or treat HAM.

The dose is appropriately set in consideration of a purpose, the severity of a disease, the age, body weight, sex, past medical history of a patient, the type of the active ingredient and the like.

### Examples

### [Example 1: Comprehensive comparative analysis for elucidation of pathogenic mechanisms and pathological conditions of HAM and adult T-cell leukemia/lymphoma (ALT)]

As shown in Figure 1, specimens of HTLV-1 nonprogressors were subjected to mRNA expression analysis as to groups, of CD4-positive cells, P, D and N classified according to CADM1 and CD7 serving as indexes for HTLV-1-infected cells.

As for HAM, CD4-positive cells and CD4-negative cells concentrated with magnetic beads from peripheral blood mononuclear cells of HAM patients were analyzed for 4 cases each. Cells were similarly separated as to HTLV-1 non-infected healthy individuals (Normal). The peripheral blood mononuclear cells are also referred to as PBMCs. For ATL (each of smoldering, chronic, and acute types of the disease), PBMCs composed mainly of CD4-positive cells were used.

One-Color Microarray-Based Gene Expression Analysis manufactured by Agilent Technologies Inc. was conducted.

As a result of comprehensive comparative analysis on nerve-related molecules, it was found that RGMa is markedly expressed in CD4-positive cells of HAM patient.

Total gene expression data were obtained from normal T-cells in 4 cases (Normal.CD4), HAM patient-derived CD4-positive T-cells in 4 cases (HAM.CD4), healthy person-derived CD4-positive/CADM1-negative/CD7-positive T-cells in 3 cases (Normal.P), HTLV-1-infected person-derived CD4-positive/CADM1-negative/CD7-positive T-cells in 5 cases (P group), HTLV-1-infected person-derived CD4-positive/CADM1-positive/CD7-positive T-cells in 5 cases (D group), HTLV-1-infected person-derived CD4-positive/CADM1-positive/CD7-negative T-cells in 5 cases (N group), acute ATL patient-derived CD4-positive/CADM1-positive/CD7-negative T-cells in 3 cases (Acute.N), healthy person-derived CD4-positive T-cells in 21 cases (Normal.CD4.1), smoldering ATL patient-derived PBMCs in 3 cases (Smoldering), chronic ATL patient-derived PBMCs in 20 cases (Chronic), and acute ATL patient-derived PBMCs in 26 cases (Acute) using Human Gene Expression 4x44K Microarray from Agilent Technologies Inc., and normalized with a median value to plot an RGMa gene level in a graph.

In the graph, Log₂ values of fluorescence intensity from the array were plotted. In the HAM patient-derived CD4-positive T-cells group, the expression level was elevated with significant difference from all the other groups (P < 0.05). The graph is shown in Figure 2.

### [Example 2: Gene expression assay in CD4-positive HAM patient]

CD4-positive T-cells were separated from PBMCs separated from the peripheral blood of five HAM patients using human CD4⁺ isolation kit (Miltenyi Biotec) and used as a cell population containing a large number of HTLV-1-infected cells. Likewise, CD4-positive T-cells were separated from PBMCs of four healthy individuals and used as a control group.

Total RNA was recovered from the separated CD4-positive T-cells, and cDNA was prepared using ReverTra Ace (Toyobo Co., Ltd.). The prepared cDNA was used to analyze difference in RGMa expression level between the HAM patient-derived CD4-positive T-cells and the healthy person-derived CD4-positive T-cells by real time PCR. 18srRNA was used as an internal control.

The analysis results are shown in a graph in Figure 3. In the graph, HD-CD4+ refers to the control group, and HAM-CD4+ refers to the HAM patient group.

### [Example 3: Analysis of RGMa-expressing cell in PBMCs]

HAM patient-derived PBMCs were treated with Fc block using Clear Back (Medical & Biological Laboratories Co., Ltd. (MBL)). Then, anti-CD3-PECy7 (Tonbo Biosciences, Inc. (TONBO)), anti-CD4-FITC (eBioscience, Inc.), and anti-CD14-PE (eBioscience, Inc.) antibodies were added thereto, and the cells were stained at 4°C for 30 minutes.

After washing of the antibody-stained PBMCs, FACS sorting was performed using Aria IIIu (Becton, Dickinson and Company(BD)) to separate and recover CD3-positive CD4-negative cells (CD3+CD4-), CD3-positive CD4-positive cells (CD3+CD4+), CD3-negative CD14-negative cells (CD3-CD14-) and CD3-negative CD14-positive cells (CD3-CD14+).

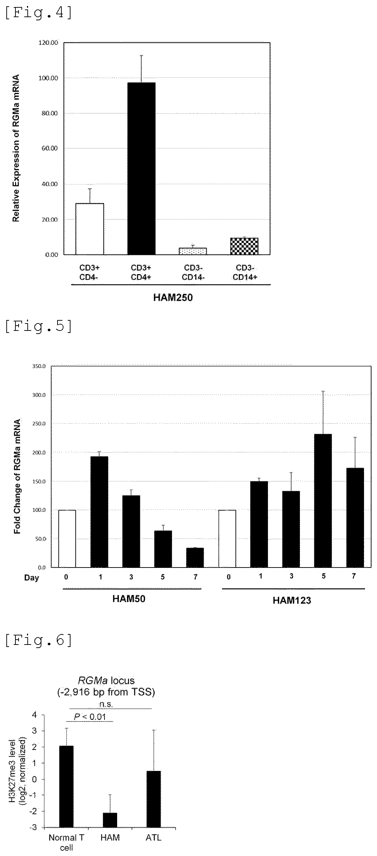

Total RNA was recovered from the recovered cells of each type, and cDNA was prepared using ReverTra Ace (Toyobo Co., Ltd.). The prepared cDNA was used to analyze difference in RGMa expression level among the cell species by real time PCR. 18s rRNA was used as an internal control. The analysis results are shown in a graph in Figure 4.

Among the HAM-PBMCs, the CD3-positive CD4-positive cells (CD3+CD4+) rich in infected cells were found to have the highest expression of RGMa.

### [Example 4: Change in RGMa expression associated with culture of HAM patient-derived PBMCs]

PBMCs of two HAM patients were suspended in a medium (RPMI1640 medium (Wako) containing 10% FBS (Gibco)), inoculated at 1e5 cells/well to 10 wells of a 96-well round bottom plate, and then cultured for 1, 3, 5 or 7 days.

Total RNA was extracted from the PBMCs of day 0 when culture was not performed as well as the PBMCs cultured for each period, and cDNA was prepared using ReverTra Ace (Toyobo Co., Ltd.). HAM patient-derived PBMCs are known to overexpress HTLV-1 virus by culture. The prepared cDNA was used to analyze change in RGMa expression associated with the culture of the HAM patient PBMCs, i.e., associated with the expression of the virus, by real time PCR. 18s rRNA was used as an internal control. The analysis results are shown in a graph in Figure 5.

### [Example 5: Analysis of H3K27me3 level on whole promoter]

An H3K27me3 level on whole promoter was obtained from normal T-cells in 3 cases (Normal T cell), HAM patient-derived CD4-positive T-cells in 4 cases (HAM), and acute ATL patient-derived PBMCs in 3 cases (ATL) using SurePrint G3 Human Promoter 2x400K Microarray from Agilent Technologies Inc., and normalized. Then, the H3K27me3 level at or around -2,916 bp upstream from the transcription start point of the RGMa gene was shown in a graph.

The graph is shown in Figure 6. In the graph, Log₂ values of fluorescence intensity from the array were shown. In the drawing, HAM50 and HAM123 respectively refer to HAM patients from which CD4-positive T-cells were obtained. It was suggested that the suppression of RGMa gene expression is removed in HAM patient-derived CD4-positive T-cells.

### [Example 6: Quantification of mRNA level of RGMa gene]

A human CD4-positive T-cell leukemia cell line Jurkat was transfected with a lentivirus vector having an insert of cDNA encoding HTLV-1 Tax. The mRNA level of the RGMa gene was measured over time for 3 days after transfection by quantitative RT-PCR. mRNA of RPL19 gene was also measured and used as an internal standard.

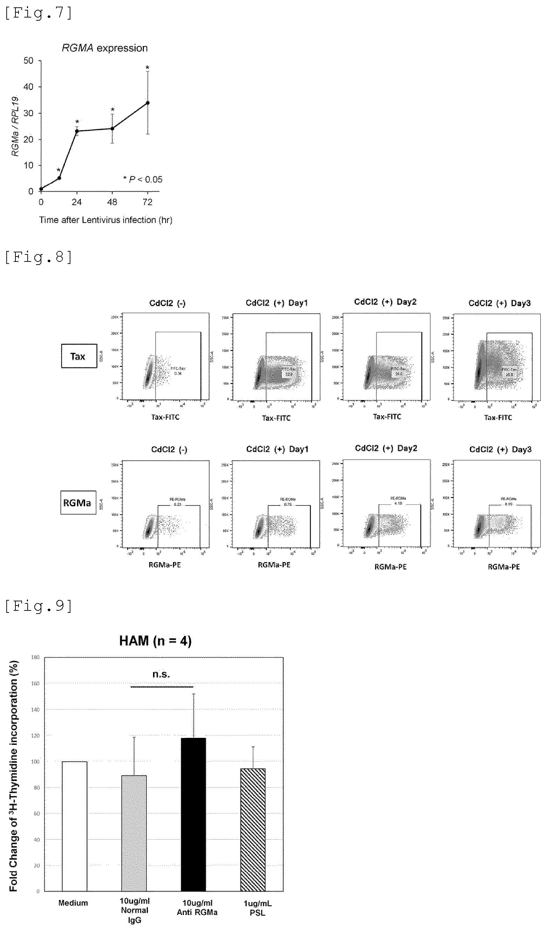

The quantitative results are shown in a graph in Figure 7. It was suggested that HTLV-1 virus causes RGMa expression.

### [Example 7: Tax-dependent induction of RGMa expression in cell line JPX-9 cell having expression of HTLV-1-tax induced]

JPX9 cells were cultured for 24 hours in a medium (RPMI1640 medium containing 10% FBS). Cadmium chloride (CdCl2; Nacalai Tesque, Inc.), which induces HTLV-1-tax expression, was added thereto with a final concentration of 20 µM, followed by culture for 1, 2, or 3 days. The cadmium chloride treated and untreated JPX9 cells were analyzed by FACS for the protein expression of Tax and RGMa.

The cadmium chloride treated and untreated JPX9 cells were each washed and permeabilized using Foxp3/Transcription Factor Staining Buffer Kit (eBioscience, Inc.). Then, an anti-Tax-FITC antibody (Lt-4; kindly provided by Professor Tanaka from University of The Ryukyus) was added thereto, and the cells were treated at 4°C for 1 hour to stain Tax protein expressed in the cells. The stained Tax protein was detected by FACS analysis using Canto II.

The cadmium chloride treated and untreated JPX9 cells were each washed. An anti-RGMa antibody (manufactured by Immuno-Biological Laboratories Co., Ltd. (IBL)) was added thereto, and the cells were treated at 4°C for 30 minutes. Then, the cells were washed, and an anti-mouse IgG-PE antibody (BioLegend, Inc.) was added thereto and reacted at 4°C for 30 minutes to stain RGMa protein expressed in JPX9. The stained RGMa protein was detected by FACS analysis using Canto II.

Figure 8 shows the results of analyzing the protein expression of Tax and RGMa.

### [Example 8: Study on effect of anti-RGMa antibody on HAM patient PBMCs]

### (Effect of anti-RGMa antibody on spontaneous proliferative activity)

PBMCs of four HAM patients were suspended in a medium (RPMI1640 medium containing 10% FBS) and inoculated at 1e5 cells/well to a 96-well round bottom plate. An anti-RGMa antibody (manufactured by R&D Systems, Inc.) was added thereto with a final concentration of 10 µg/ml and total volume of 0.1 ml of the culture solution, and the cells were cultured at 37°C under 5% CO₂ conditions for 7 days in the culture solution.

A non-supplemented group (Medium), a group supplemented with the same concentration of Normal Goat IgG (Santa Cruz Biotechnology, Inc.) (Normal IgG), and a group supplemented with 1 µg/ml prednisolone (PSL) (Funakoshi Co., Ltd.) were used as controls.

After 6 days from the start of culture, 1 µ Ci ³H-thymidine was added to each well, and the cells were cultured at 37°C under 5% CO₂ conditions for 16 hours. Then, the cultured cells were adsorbed to a glass filter (Printed Filtermat A, PerkinElmer Inc.) using a cell harvester (Tomtec MH3, PerkinElmer Inc.) and dried. Then, the filter was impregnated with solid scintillator Meltilex-A (PerkinElmer Inc.). The amount of ³H-thymidine taken up into the cells was measured using MicroBeta (WALLAC MicroBeta TriLux 1450-021). An average of the counts of ³H-thymidine in the Medium group of HAM patient PBMCs was defined as 100%. A relative value of each group was calculated to determine an average ³H-thymidine incorporation rate for the four HAM patients. The results are shown in Figure 9.

### (Effect of anti-RGMa antibody on change in amount of HTLV-1 provirus)

PBMCs of four HAM patients were suspended in a medium (RPMI1640 medium containing 10% FBS) and inoculated at 1e5 cells/well to a 96-well round bottom plate. An anti-RGMa antibody (manufactured by R&D Systems, Inc.) was added thereto with a final concentration of 10 µg/ml and total volume of 0.1 ml of the culture solution, and the cells were cultured at 37°C under 5% CO₂ conditions for 7 days in the culture solution.

A non-supplemented group (Medium), a group supplemented with the same concentration of Normal Goat IgG (Santa Cruz Biotechnology, Inc.) (Normal IgG), and a group supplemented with 1 µg/ml prednisolone (PSL) (Funakoshi Co., Ltd.) were used as controls.

After 7 days from the start of culture, genomic DNA was extracted from masses of the cells from which a supernatant was removed by centrifugation. The extracted genomic DNA was used to measure the amount of HTLV-1 provirus (proportion of infected cells) by real time PCR.

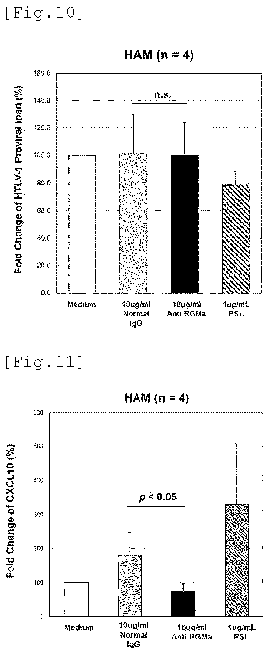

The amount of HTLV-1 provirus in the Medium group of HAM patient PBMCs was defined as 100%. A relative value of the amount of HTLV-1 provirus in each group was calculated to determine an average amount of HTLV-1 provirus of the four HAM patients. The results are shown in Figure 10.

### (Effect of anti-RGMa antibody on CXCL10 production)

In order to analyze the effect of an anti-RGMa antibody on CXCL10 production in HAM patient PBMCs, PBMCs of four HAM patients were suspended in a medium (RPMI1640 medium containing 10% FBS) and inoculated at 1e5 cells/well to a 96-well round bottom plate. An anti-RGMa antibody (manufactured by R&D Systems, Inc.) was added thereto with a final concentration of 10 µg/ml and total volume of 0.1 ml of the culture solution, and the cells were cultured at 37°C under 5% CO₂ conditions for 7 days in the culture solution.

A non-supplemented group (Medium), a group supplemented with the same concentration of Normal Goat IgG (Santa Cruz Biotechnology, Inc.) (Normal IgG), and a group supplemented with 1 µg/ml prednisolone (PSL) (Funakoshi Co., Ltd.) were used as controls.

After 7 days from the start of culture, the culture solution was centrifuged, and only the culture supernatant was recovered. The concentration of CXCL10 in the culture supernatant was measured by flow cytometer FACS Canto II (BD Biosciences) using Cytokine Beads Array kit (BD Biosciences).

The CXCL10 concentration in the culture solution of the Medium group was defined as 100%. A relative value of the CXCL10 concentration in the culture solution of each group was calculated to determine an average CXCL10 concentration of the four HAM patients. The results are shown in Figure 11.

### (Effect of anti-RGMa antibody on cytokine production of HAM patient PBMCs)

In order to analyze the effect of an anti-RGMa antibody on the production of various cytokines in HAM patient PBMCs, PBMCs of four HAM patients were suspended in a medium (RPMI1640 medium containing 10% FBS) and inoculated at 1e5 cells/well to a 98-well round bottom plate. An anti-RGMa antibody (manufactured by R&D Systems, Inc.) was added thereto with a final concentration of 10 µg/ml and total volume of 0.1 ml of the culture solution, and the cells were cultured at 37°C under 5% CO₂ conditions for 7 days in the culture solution.

A non-supplemented group (Medium), a group supplemented with the same concentration of Normal Goat IgG (Santa Cruz Biotechnology, Inc.) (Normal IgG), and a group supplemented with 1 µg/ml prednisolone (PSL) (Funakoshi Co., Ltd.) were used as controls.

After 7 days from the start of culture, the culture solution was centrifuged, and only the culture supernatant was recovered. The concentrations of IFNγ, TNF, IL-2, and IL-10 in the culture supernatant were measured by flow cytometer FACS Canto II (BD Biosciences) using Cytokine Beads Array kit (BD Biosciences).

The concentration of each cytokine in the culture solution of the Medium group was defined as 100%. A relative value of the cytokine concentration under each culture condition was calculated to determine an average value of the four HAM patients.

### [Example 9: Induction of apoptosis of neuronal cell line by HAM-PBMCs]

A neuronal cell line NB-1 or SK-N-AS was inoculated to a 6-well plate and cultured for 24 hours. Then, healthy individual (HD) or HAM patient PBMCs were added thereto and cocultured. After 48 hours from the start of coculture, the added PBMCs were removed together with the medium. After washing with PBS, the neuronal cell line was recovered.

Each of the recovered neuronal cell lines was analyzed by the TUNEL method (MEBSTAIN Apoptosis TUNEL Kit Direct (MBL)), which specifically detects cells in which DNA fragmentation occurs by apoptosis. The analysis results are shown in Figure 13. The X axis in the histogram corresponds to the intensity of DNA fragmentation positivity. The HAM-derived cells strongly induced the apoptosis of the neuronal cell line as compared with the HD-derived cells.

Specifically, cell death was analyzed according to the following <Experimental procedure>.

### <Experimental procedure>

The neuronal cell lines NB-1 and SK-N-AS were each inoculated to a 6-well plate and cultured for 24 hours.

Subsequently, HD- or HAM-PBMCs were added thereto (in an amount of two times the number of the neuronal cell line inoculated) and cultured for 48 hours.

Then, the cells were fixed using 4% paraformaldehyde and permeabilized with 70% ethanol.

In order to perform DNA nick end labeling, the cells were suspended in 20 uL of TdT solution (TdT buffer II : TdT : FITC-dUTP = 18 : 1 : 1) of MEBSTAIN Apoptosis TUNEL Kit Direct (MBL) and reacted at 37°C for 60 minutes, followed by FACS analysis.

### [Example 10: Suppressive effect of anti-RGMa antibody on apoptosis of neuronal cell line induced by HTLV-1 Taxexpressing T-cell line]

A neuronal cell line NB-1 was inoculated to a 6-well plate and cultured for 24 hours. Then, unstimulated JPX9 (JPX9(-)) or JPX9 having Tax expression induced by the addition of 20 µM cadmium chloride for 24 hours (JPX9(+CdCl2)) was added thereto and cocultured. Also, Normal Mouse IgG2b (MBL) or an anti-RGMa antibody (IBL) was added to the cocultures of the NB-1 cells and (JPX9(+CdCl₂) with a final concentration of 10 µg/ml. After 48 hours from the start of coculture, the added JPX9 was removed together with the medium. After washing with PBS, the neuronal cell line was recovered. Each of the recovered neuronal cell line was analyzed by the TUNEL method (MEBSTAIN Apoptosis TUNEL Kit Direct (MBL)), which specifically detects cells in which DNA fragmentation occurs by apoptosis. The analysis results are shown in Figure 14. The X axis in the histogram corresponds to the intensity of DNA fragmentation positivity.

Specifically, cell death was analyzed according to the following <Experimental procedure>.

### <Experimental procedure>

The neuronal cell line NB-1 was inoculated to a 6-well plate and cultured for 24 hours.

Subsequently, JPX9(-) or JPX9(+CdCl₂) was added to the NB-1 cells and cocultured. The number of JPX9(-) or JPX9(+CdCl₂) cells was set to an amount of two times the number of NB-1 cells inoculated. JPX9(+CdCl₂) was washed three times with 10 ml of a medium for the removal of cadmium chloride and then added to the NB-1 cells.

Subsequently, Normal Mouse IgG2b (MBL) or an anti-RGMa antibody (IBL) was added to the cocultures of the NB-1 cells and (JPX9(+CdCl₂) with a final concentration of 10 µg/ml and cultured for 48 hours.

Then, the cells were fixed using 4% paraformaldehyde, permeabilized with 70% ethanol., and stained by the addition of an anti-CD45-V450 antibody.

In order to perform DNA nick end labeling, the cells were suspended in 20 uL of TdT solution (TdT buffer II : TdT : FITC-dUTP = 18 : 1 : 1) of MEBSTAIN Apoptosis TUNEL Kit Direct (MBL) and reacted at 37°C for 60 minutes, followed by FACS analysis.

## Claims

1. A therapeutic or prophylactic agent for use in treating HTLV-1-associated myelopathy (HAM), wherein the agent comprises an RGMa-inhibiting substance,
wherein the RGMa-inhibiting substance is an antibody recognizing RGMa.

2. The therapeutic or prophylactic agent for the use according to claim 1, wherein a pharmacologically effective amount of an RGMa-inhibiting substance is to be administered to an HAM patient in need thereof.

## Patentansprüche

1. Therapeutisches oder prophylaktisches Mittel zur Verwendung bei der Behandlung von HTLV-1-assoziierter Myelopathie (HAM), wobei das Mittel eine RGMa-hemmende Substanz umfasst,
wobei die RGMa-hemmende Substanz ein RGMa-erkennender Antikörper ist.

2. Therapeutisches oder prophylaktisches Mittel zur Verwendung nach Anspruch 1, wobei eine pharmakologisch wirksame Menge einer RGMa-hemmenden Substanz an einen HAM-Patienten, der diese benötigt, zu verabreichen ist.

## Revendications

1. Agent thérapeutique ou prophylactique pour être utilisé dans le traitement de la myélopathie associée au HTLV-1 (HAM), dans lequel l'agent comprend une substance inhibant la RGMa,
dans lequel la substance inhibant la RGMa est un anticorps reconnaissant la RGMa.

2. Agent thérapeutique ou prophylactique pour être utilisé selon la revendication 1, dans lequel une quantité pharmacologiquement efficace d'une substance inhibant la RGMa doit être administrée à un patient HAM qui en a besoin.
